(19) **Europäisches Patentamt / European Patent Office / Office européen des brevets**

(11) **EP 2 402 018 A1**

(12) **EUROPEAN PATENT APPLICATION**
published in accordance with Art. 153(4) EPC

(43) Date of publication:
**04.01.2012 Bulletin 2012/01**

(21) Application number: **10746296.2**

(22) Date of filing: **19.02.2010**

(51) Int Cl.:
*A61K 33/08* (2006.01)　　*A61K 9/14* (2006.01)
*A61K 47/10* (2006.01)　　*A61K 47/38* (2006.01)
*A61P 1/10* (2006.01)　　*A61P 3/02* (2006.01)

(86) International application number:
**PCT/JP2010/053021**

(87) International publication number:
**WO 2010/098417 (02.09.2010 Gazette 2010/35)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.02.2009 JP 2009042795**

(71) Applicant: **KYOWA CHEMICAL INDUSTRY CO., LTD.**
**Kagawa-ken 761-0113 (JP)**

(72) Inventors:
• **KITAJIMA Hideaki**
  **Kita-gun**
  **KAGAWA 761-0705 (JP)**
• **KAWANABE Naruhito**
  **Kita-gun**
  **KAGAWA 761-0705 (JP)**

(74) Representative: **Benson, John Everett et al**
**J. A. Kemp & Co.**
**14 South Square**
**Gray's Inn**
**London WC1R 5JJ (GB)**

(54) **MAGNESIUM OXIDE FINE GRANULES**

(57) Magnesium oxide granules which are excellent in dissolution property, are palatable, rarely leave any rough feeling in the oral cavity and have a good color in appearance.

The granules comprise magnesium oxide particles which are represented by the following formula (1) and have an average secondary particle diameter of 0.1 to 25 $\mu$m and an apparent specific volume of 3 to 20 ml/g, a sugar alcohol and a disintegrating agent.

$$(Mg^{2+}_{1-x}Zn^{2+}_{x}) O \qquad (1)$$

(In the formula, X is a numeral of 0 to 0.02.)

Fig. 1

Dissolution test

Dissolution rate(%) vs Elapsed time

Sample A　　Sample X

Printed by Jouve, 75001 PARIS (FR)

**Description**

TECHNICAL FIELD

The present invention relates to granules which comprise magnesium oxide as an effective ingredient and are useful as a laxative agent and nutritional food.

BACKGROUND ART

Although magnesium oxide granules are available on the market mainly as a laxative agent, all commercially available magnesium oxide granules taste bad (smelling of mud), and agglomerated particles remain in the oral cavity, thereby giving a persistent rough feeling on the tongue. The color of the granules is achromatic and dull in appearance, and the granules have a defect such as poor dissolution property.

Patent Document 1 proposes granules having an average particle diameter of not more than 15 $\mu$m obtained by uniformly dispersing a sugar alcohol and a disintegrating agent into an inorganic antacid, and spraying and drying the resulting suspension (suspension wet granulation drying method). However, the granules have defects such as the particles are susceptible to moisture (water), greatly change with the passage of time, deteriorate quickly and have a long disintegration time (2 to 3 minutes).

Patent Document 2 discloses a magnesium oxide laxative agent containing a sugar alcohol. The magnesium oxide particles used as a laxative agent are a mixture of granules consisting mainly of particles that do not pass through a 75 $\mu$m sieve and granules consisting mainly of particles that pass through a 75 $\mu$m sieve, give an unpleasant feeling on the tongue and have poor dissolution property due to their large particle diameters.

(Patent Document 1) JP-A 10-120554
(Patent Document 2) JP-A 2008-115058

DISCLOSURE OF THE INVENTION

It is an object of the present invention to provide magnesium oxide granules have excellent dissolution property, are palatable, do not leave any rough feeling in the oral cavity and have a good color in appearance.

The inventors of the present invention have conducted intensive studies on magnesium oxide particles which are excellent in dissolution property, taste and feeling on the tongue and have suitable strength. As a result, they have found that granules which do not leave any rough feeling in the oral cavity and quickly dissolve are obtained by granulating magnesium oxide particles having a small average secondary particle diameter and a specific apparent specific volume with a sugar alcohol and a disintegrating agent. The present invention has been accomplished based on this finding.

That is, the present invention is granules which comprise magnesium oxide particles having an average secondary particle diameter of 0.1 to 25 $\mu$m and an apparent specific volume of 3 to 20 ml/g and represented by the following formula (1), a sugar alcohol and a disintegrating agent.

$$(Mg^{2+}_{1-x}Zn^{2+}_{x})O \qquad (1)$$

(In the formula, X is a numeral of 0 to 0.02.)

BRIEF DESCRIPTION OF THE DRAWINGS

Fig. 1 shows the results of a dissolution test on the sample A of Example 1 and a comparative sample X.
Fig. 2 shows the dissolution states of these samples for the dissolution test one minute after injection.

BEST MODE FOR CARRYING OUT THE INVENTION

(Magnesium oxide particles)

The magnesium oxide particles constituting the granules of the present invention are represented by the following formula (1).

$$(Mg^{2+}_{1-x}Zn^{2+}_{x})O \qquad (1)$$

In the formula, X is a numeral of 0 to 0.02. Magnesium oxide particles in which X is more than "0" are not a mixture of magnesium oxide and zinc but contain a zinc atom in the crystal structure of magnesium oxide and have the same crystal structure as that of magnesium oxide. The magnesium oxide particles show the same diffraction pattern as that of magnesium oxide according to a powder X-ray diffraction method. X is 0 to 0.02, preferably 0.001 to 0.015, more preferably 0.005 to 0.01. When X becomes large, it is possible that the amount of Zn including Zn ingested from food may exceed the required amount as an essential mineral.

The average secondary particle diameter measured by a laser diffraction scattering method of the magnesium oxide particles is 0.1 to 25 $\mu$m, preferably 0.5 to 20 $\mu$m, more preferably 0.5 to 18 $\mu$m. When the particle diameters of the magnesium oxide particles are large, the disintegration time becomes long and the magnesium oxide particles are not quickly dispersed in the oral cavity.

The apparent specific volume of each of the magnesium oxide particles is 3 to 20 ml/g, preferably 4 to 15 ml/g. When the apparent specific volume of the magnesium oxide particle is smaller than 3 ml/g, the dissolution property degrades. When the apparent specific volume is larger than 20 ml/g, the bulk becomes large and granulation becomes difficult.

The magnesium oxide particles are produced by calcine magnesium hydroxide particles. The magnesium hydroxide particles can be produced by precipitating a magnesium ion contained in sea water or bittern as magnesium hydroxide with an alkali. Examples of the alkali include calcium hydroxide, caustic soda, potassium hydroxide, lithium hydroxide and ammonia water. Out of these, caustic soda or calcium hydroxide is preferred as an alkali source.

The precipitated magnesium hydroxide is preferably heated at 100 to 120˚C. When magnesium hydroxide particles having an average secondary particle diameter of 0.1 to 25 $\mu$m are calcined at 500 to 1,000˚C, magnesium oxide having an average secondary particle diameter of 0.1 to 25 $\mu$m is obtained. Magnesium oxide having an apparent specific volume of 3 to 20 ml/g is selected from the obtained magnesium oxide. Calcining is carried out at preferably 500 to 1, 000˚C, more preferably 600 to 900˚C for 0.1 to 10 hours. Long-term calcining at a high temperature makes magnesium oxide particles hard and the disintegration of the particles difficult.

Magnesium oxide particles prepared by containing zinc (Zn) as a solid solution in magnesium oxide can be produced by adding an alkaline substance to an aqueous solution containing a magnesium ion and a zinc (Zn) ion in almost the same as or smaller than the total amount of these cations and reacting them with each other under agitation. The obtained reaction product may be optionally hydrothermally processed in an autoclave at 100 to 200˚C. Thereafter, the reaction product can be prepared by using commonly used means such as rinsed in water, dehydrated, dried, calcined, ground and classified properly as they are used in magnesium oxide. It is preferred to use magnesium nitrate or magnesium chloride as a supply source for the magnesium ion. It is also preferred to use zinc nitrate or zinc chloride as a supply source for the zinc (Zn) ion. Sodium hydroxide is preferred as the alkaline substance.

(Sugar alcohol)

Examples of the sugar alcohol used in the present invention include xylitol, erythritol, sorbitol and mannitol. Out of these, mannitol is preferred. The amount of the sugar alcohol is preferably 5 to 10 parts by mass, more preferably 5 to 9 parts by mass, much more preferably 6 to 8 parts by mass based on 100 parts by mass of magnesium oxide.

(Disintegrating agent)

Examples of the disintegrating agent include starch (for example, corn starch), cross povidone, hydroxypropyl cellulose having a low degree of substitution, cross carmellose sodium, carmellose calcium and carmellose. These disintegrating agents may be used in a combination of two or more, and cross povidone, carmellose calcium and carmellose are particularly preferred. The most preferred disintegrating agent is carmellose. The amount of the disintegrating agent is preferably 1 to 10 parts by mass, more preferably 1 to 7 parts by mass, much more preferably 1 to 3 parts by mass based on 100 parts by mass of magnesium oxide.

<Granules production method>

The granules of the present invention can be produced by mixing together magnesium oxide particles, a sugar alcohol and a disintegrating agent to prepare a mixture and dry granulating the mixture. The content of the magnesium oxide particles represented by the above formula (1) in the mixture is preferably not less than 80 mass%, more preferably 85 to 95 mass%. Mixing is carried out by using a container type, V-shaped or W-shaped mixer.

Granulation is preferably carried out at a low pressure by using a dry granulating machine. The roll pressure in this case is preferably 3 to 12 MPa, more preferably 4 to 8 MPa. The granular particles are obtained from sheet-like molded article by using an oscillator type grinder. The opening of a screen set in the oscillator is preferably 0.7 to 1.2 mm, more preferably 0.8 to 1.0 mm. Granulation particles having an average particle diameter of 0.25 to 0.45 mm and a bulk density of 0.5 to 0.7 g/mL are thus obtained.

The obtained magnesium oxide granular particles are classified by using a vibrating sieve (0.15 mm, 0.5 mm) to produce granules having an average particle diameter of 0.2 to 0.4 mm, preferably 0.25 to 0.35 mm.

The bulk density of the granules is preferably 0.4 to 0.7 g/ml, more preferably 0.5 to 0.65 g/ml, much more preferably 0.5 to 0.6 g/ml.

As for the particle size distribution of the granules, the content of particles having a particle diameter of less than 500 $\mu$m to not less than 355 $\mu$m is preferably 30 to 45 mass%, more preferably 32 to 42 mass%. The content of particles having a particle diameter of less than 355 $\mu$m to not less than 180 $\mu$m is preferably 40 to 50 mass%, more preferably 40 to 49 mass%. The content of particles having a particle diameter of less than 180 $\mu$m to not less than 150 $\mu$m is preferably 10 to 28 mass%, more preferably 10 to 27 mass%.

The obtained granules are preferably mixed with flavoring powders in a container or mixer. Examples of the flavoring powder include peppermints, L-menthol, orange powders and strawberry essence. A trace amount of peppermint powder or L-menthol is adsorbed to hydrous silicon dioxide to enhance the palatability of the granules.

The present invention includes a method of using magnesium oxide particles which are represented by the following formula (1) and have an average secondary particle diameter of 0.1 to 25 $\mu$m and an apparent specific volume of 3 to 20 ml/g as magnesium oxide particles for a laxative agent containing magnesium oxide, a sugar alcohol and a disintegrating agent.

$$(Mg^{2+}_{1-x}Zn^{2+}_{x})O \qquad (1)$$

(In the formula, X is a numeral of 0 to 0.02.)

The present invention also includes a method of improving the feeling of a laxative agent containing magnesium oxide, a sugar alcohol and a disintegrating agent on the tongue, wherein magnesium oxide particles which are represented by the following formula (1) and have an average secondary particle diameter of 0.1 to 25 $\mu$m and an apparent specific volume of 3 to 20 ml/g are used as the magnesium oxide.

$$(Mg^{2+}_{1-x}Zn^{2+}_{x})O \qquad (1)$$

(In the formula, X is a numeral of 0 to 0.02.)

Examples

The following examples are given to further illustrate the present invention. In the examples, physical properties were measured by the following methods.

(a) Analysts of MgO, Zn, D-mannitol, crystalline cellulose, carmellose, hydrous silicon dioxide, peppermint and L-menthol

These were measured by an atomic absorption method.

(b) Particle size distribution and average secondary particle diameter of magnesium oxide particles

They were measured by using the MIKROTRAC particle size distribution meter of SPA type (of LEEDS & NORTHRUP INSTRUMENTS).

700 mg of sample powders was added to 70 mL of water and then processed ultrasonically (MODEL US-300 of NISSEI Co., Ltd., current of 300 $\mu$A) for 3 minutes, 2 to 4 mL of the resulting dispersion was collected and added to the sample chamber of the above particle size distribution meter containing 250 mL of deaerated water, the analyzing meter was activated to circulate the suspension for 8 minutes, and then the particle size distribution was measured. The measurement was carried out twice in total to calculate the arithmetic mean value of 50 % cumulative secondary particle diameters obtained from these measurements as the average secondary particle diameter of the sample.

(c) Apparent specific volume of magnesium oxide particles

This was measured in accordance with JIS K5101.

(i) An apparent specific volume meter was leveled off. The dropping funnel of a funnel support was set on the meter. A sieve was placed on the funnel. Thereafter a receiver was placed on a receiver support properly.
(ii) One spoon of the sample was placed on the sieve and swept lightly over the front surface of the sieve uniformly with a brush, and a sample passing through the sieve was received on the receiver and was regarded as processed sample.

(iii) This operation was repeated until the processed sample accumulated like a mountain on the receiver.

(iv) The mountain portion was scraped off with a spatula.

(v) The mass of the content of the receiver was weighed. Note: Vibration should not be applied to the receiver during the operations (ii) to (iv).

The apparent specific volume was calculated to two places of decimals from the following equation.

$$G = V/F$$

G: apparent specific volume (mL/g)
F: mass of processed sample in receiver (g)
V: volume of receiver (mL)

(d) Bulk density

This was measured in accordance with Determination of Bulk and Tapped Densities measuring method (Method 2, (Constant volume method)) which is one of the Powder Property Determinations out of the General Test Processes and Apparatus of The Japanese Pharmacopoeia fifteenth edition.

$$\rho B = (Mt-M0)/V$$

$\rho B$: bulk density measured by constant volume method (g/mL)
Mt: total mass of powders and measurement vessel (g)
M0: mass of measurement vessel (g)
V: volume of measurement vessel (mL)

(e) Dissolution test

This was carried out in accordance with Puddle Method which is one of the general test methods of 15-th revised Japanese Pharmacopoeia.

(f) Disintegration Test

This was conducted by a disintegration test method which is one of the general test methods of The Japanese Pharmacopoeia fifteenth edition (g) Method of measuring particle size distribution and method of calculating average particle diameter of granules

They were measured by using OCTAGON DIGITAL (of Endecotts Ltd.) and JIS standard sieves (150 $\mu$m, 180 $\mu$m, 355 $\mu$m, 500 $\mu$m). A sample of 100 g was processed continuously at a vibration intensity of 5 with OCTAGON DIGITAL for 5 minutes to calculate the particle size distribution of the sample from the weight of each fraction. The average particle diameter (X50) was calculated from the obtained particle size distribution by a weighted average method.

Preparation Example 1 (magnesium oxide particles "a")

Magnesium hydroxide (magnesium hydroxide for Magmitt tablets (MgO) manufactured by Kyowa Chemical Industry Co., Ltd.) was calcined at 800°C for 2 hours to obtain magnesium oxide "a" to be used in Example 1.

Preparation Example 2 (magnesium oxide particles "b")

Magnesium oxide "b" to be used in Example 2 was obtained in the same manner as in Preparation Example 1 except that the calcinning temperature was changed to 700°C.

Preparation Example 3 (magnesium oxide particles "c")

A mixed solution of magnesium nitrate and zinc nitrate (1.50 mol/L of magnesium nitrate, 1.5 x $10^{-3}$ mol/L of zinc nitrate, designated as solution A) and a 6.5 N aqueous solution of sodium hydroxide (designated as solution B) were continuously injected into a reaction tank containing water under agitation by using a metering pump. The residence time of the reaction solution in the reaction tank was 30 minutes at a reaction temperature of 40°C and a reaction pH of 10.5, and 700 mL of the reaction suspension overflown from the reaction tank was transferred to an autoclave and subjected to a hydrothermal reaction at 100°C for 3 hours. After the reaction product was cooled, it was separated by filtration, rinsed in water, dried at 110°C for 24 hours, ground and put through a sieve to obtain magnesium hydroxide particles.

The magnesium hydroxide particles were calcined at 700°C for 2 hours in a baking furnace to obtain magnesium oxide particles "c" to be used in Example 3.

Preparation Example 4 (magnesium oxide particles "d")

Magnesium oxide "d" to be used in Example 4 was obtained in the same manner as in Preparation Example 3 except that the solution A was changed to a mixed solution of 1.30 mol/L of magnesium nitrate and 1.31 x $10^{-2}$ mol/L of zinc nitrate.

Examples 1 to 4

A compressed platy crystal was granulated from four different kinds of magnesium oxide particles shown in Table 1 and components shown in Table 2 in a ratio shown in Table 2 by using a roller compactor (RC156 of Furointo Sangyo Co., Ltd.). The granulation conditions are shown in Table 3. Granular particles were obtained from the obtained flake by using an oscillator (manufactured by Furointo Sangyo Co., Ltd.). The granular particles were classified by a vibration sieve (circular (ultrasonic) sieve of Kowa Kogyosho Co., Ltd.) to produce granules. The granules were mixed with flavoring powders obtained by mixing together hydrous silicon dioxide and a trace amount of peppermint powder by means of a container type mixer so as to obtain granules having a diameter of about 0.3 mm. The physical properties of the obtained granules are shown in Table 4.

Table 1

| Magnesium oxide particles | a | b | c | d |
|---|---|---|---|---|
| Composition | MgO | MgO | $Mg_{0.999}Zn_{0.001}O$ | $Mg_{0.990}Zn_{0.010}O$ |
| Content of Zn(wt%) | 0 | 0 | 0.16 | 1.59 |
| Average secondary particle diameter ($\mu$m) | 11 | 16 | 11 | 0.52 |
| Apparent specific volume(ml/g) | 5 | 7 | 6 | 11 |

Table 2

| | Example 1 | Example 2 | Example 3 | Example 4 |
|---|---|---|---|---|
| Granules sample | A | B | C | D |
| Type of magnesium oxide Mass% | a | b | c | d |
| | 1040g(86.66) | 1040g(86.66) | 1040g(86.66) | 1040g(86.66) |
| D-mannitol (Mannit P) | 77.5g(6.46) | 77.5g(6.46) | 77.5g(6.46) | 77.5g(6.46) |
| Carmellose (NS-300) | 77.5g(6.45) | 77.5g(6.46) | 77.5g(6.46) | 77.5g(6.46) |
| Hydrous silicon dioxide (Carplex #67) | 5g(0.42) | 5g(0.42) | 5g(0.42) | 5g(0.42) |
| Peppermint (peppermint powder) | trace amount | trace amount | trace amount | trace amount |
| Total | 1200g | 1200g | 1200g | 1200g |
| The figures within the parentheses indicate mass. | | | | |

Table 3

| Item | Condition |
|---|---|
| Roll pressure (MPa) | 5 |
| Roll clearance (mm) | 0.9 |
| Roll revolution (rpm) | 15 |
| Screw revolution (rpm) | 36 |
| Oscillator (rpm) | 80 |

Table 4

| | Ex. 1 | Ex.2 | Ex.3 | Ex.4 |
|---|---|---|---|---|
| Granules sample | A | B | C | D |
| Bulk density (g/ml) | 0.56 | 0.56 | 0.60 | 0.56 |
| Particle size distribution (less than 500 $\mu$m to not less than 355 $\mu$m) (%) | 40.27 | 33.12 | 34.54 | 40.32 |
| Less than 355 $\mu$m to not less than 18 0 $\mu$m (%) | 48.81 | 40.89 | 42.55 | 48.47 |
| Less than 180 $\mu$m to not less than 150 $\mu$m (%) | 10.92 | 25. 99 | 22. 91 | 11.21 |
| Average particle diameter (X50)(mm) particle diameter | 0.32 | 0.28 | 0.29 | 0.32 |
| Ex.: Example | | | | |

<Disintegration test>

The sample A of Example 1 and commercially available magnesium oxide granules manufactured by Yoshida Pharmaceutical Co., Ltd. (trade name: Maglax granules) (sample X) as Comparative Example 1 were used for test by a disintegration test method which is one of the general test methods of The Japanese Pharmacopoeia fifteen edition. The results are shown in Table 5 . The sample X contained magnesium oxide (83.30 mass%) and hydroxymethyl cellulose (16.73 mass%).

Table 5

| | Dissolution rate (%) | |
|---|---|---|
| | Example 1 | Comparative Example 1 |
| Time (minutes) | Sample A | Sample X |
| 0 | 0 | 0 |
| 5 | 27.93 | 14.8 |
| 10 | 34.10 | 20.2 |
| 15 | 41.70 | 23.9 |
| 30 | 54.97 | 31.9 |
| 60 | 74.00 | 46.2 |
| 90 | 83.33 | 55.8 |
| 120 | 88.10 | 64.6 |
| Testing solution: disintegration test solution I Puddle revolution: 50 rpm | | |

<Dissolution test>

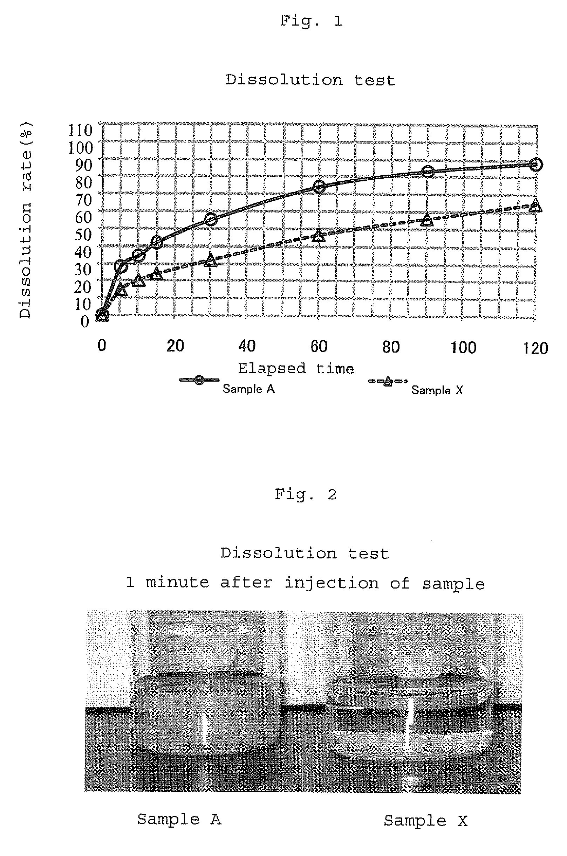

A dissolution test was made on the sample A obtained in Example 1. A dissolution test was made on the magnesium oxide granules (sample X) manufactured by Yoshida Pharmaceutical Co., Ltd. as a comparative example. The results are shown in Fig. 1. It is understood that the magnesium oxide granules of the present invention show excellent dissolution. Fig. 2 shows a dissolution state 1 minute after the injection of the sample.

Example 5

A sample A-2 was prepared in the same manner as the sample A except that the peppermint was changed to L-menthol.

Example 6

A sample C-2 was prepared in the same manner as the sample C except that the peppermint was changed to L-menthol.

<Sensory tests for dissolution property, taste and feeling on tongue>

About 1.2 g of each of the samples A, A-2, B, C, C-2 and D and the comparative sample X was administered to 5 healthy adult men and 5 healthy adult women together with water to carry out sensory tests such as dissolution property, taste and feeling on the tongue in the oral cavity. As shown in the results of Table 6, the magnesium oxide granules of the present invention which comprised peppermint as a flavoring agent were excellent.

Table 6 Results of sensory tests

|  | Ex.1 | Ex.5 | Ex.2 | Ex.3 | Ex.6 | C.Ex.1 | Ex.4 |
|---|---|---|---|---|---|---|---|
|  | A | A-2 | B | C | C-2 | X | D |
| Dissolution property | ○ | ○ | ○ | ○ | ○ | × | ○ |
| Taste | ◎ | ○ | ◎ | Δ | Δ | × | ◎ |
| Feeding on the tongue | ◎ | ◎ | ◎ | ◎ | ◎ | Δ | ◎ |
| Rating | 1 | 4 | 1 | 5 | 6 | 7 | 1 |
| ◎: 9 to 10, out of 10, who felt satisfactory<br>○: 6 to 8, out of 10, who felt satisfactory<br>Δ: 3 to 5, out of 10, who felt satisfactory<br>× : 0 to 2, out of 10, who felt satisfactory | | | | | | | |

Example 7

15 people who had been constipated for 3 days or more were divided into 3 groups. Magnesium oxide granules A obtained in Example 1 of the present invention were administered to the first group after supper. Magnesium oxide granules D obtained in Example 4 were administered to the second group after supper, 2 g of placebo granules were administered to each person of the third group after supper. The laxative effects of these samples were evaluated from the defecation state of each person for 12 hours after administration.
The results are shown below. The figures within the parentheses indicate ages.

| First group | Second group | Third group |
|---|---|---|
| ♂ (56) Diarrhea | ♂ (54) loose feces ♀ (57) | No defecation |
| ♀ (49) loose feces | ♂ (46) loose feces ♀ (49) | Hard feces |
| ♀ (41) loose feces ♀ (39) | Diarrhea | ♀ (42) No defecation |
| ♀ (35) loose feces ♀ (35) | loose feces ♀ (36) | Hard feces |
| ♀ (26) loose feces ♀ (25) | loose feces ♀ (28) | No defecation |

Effect of the Invention

The granules of the present invention are excellent in dissolution property, taste and feeling on the tongue and have appropriate strength. What are important as the quality of magnesium oxide granules are dosing properties (taste and feeling on the tongue), dissolution property and strength. Although it is essential to blend a binder so as to provide strength to the granules, when the binder is blended, the dissolution rate is generally reduced. The granules of the present invention are characterized by comprising magnesium oxide particles having a specific particle diameter and a specific apparent specific volume as a design for attaining the improvement of dissolution rate the maintenance of appropriate strength that does not cause a problem in the distribution of general medical suppliers. The dissolution rate of conventional magnesium oxide granules is not more than 30 % after 15 minutes and not more than 70% after 2 hours when magnesium oxide remains in the stomach. The dissolution rate of the magnesium oxide granules of the present invention is 60% after 15 minutes and almost 100 % after 2 hours.

Since the granules of the present invention contain magnesium oxide particles having a specific particle diameter and a specific apparent specific volume, they rarely leave any rough feeling in the oral cavity. That is, the magnesium oxide granules of the present invention are superior to conventional products in dissolution rate, taste and feeling on the tongue.

The granules of the present invention which comprise magnesium oxide particles obtained by containing a specific amount of zinc (Zn) as a solid solution in magnesium oxide as a constituent component rarely damage the mucosal membrane of the stomach lining and have excellent ulcer healing action.

**Claims**

1. Granules comprising magnesium oxide particles which are represented by the following formula (1) and have an average secondary particle diameter of 0.1 to 25 $\mu$m and an apparent specific volume of 3 to 20 ml/g, a sugar alcohol and a disintegrating agent.

$$(Mg^{2+}_{1-x}Zn^{2+}_{x})O \qquad (1)$$

(In the formula, X is a numeral of 0 to 0.02.)

2. The granules according to claim 1, wherein X in the formula (1) is 0.0005 to 0.01.

3. The granules according to claim 1, wherein the content of the magnesium oxide particles is 80 to 95 mass%.

4. The granules according to claim 1, wherein the content of the sugar alcohol is 5 to 10 parts by mass based on 100 parts by mass of the magnesium oxide particles.

5. The granules according to claim 1, wherein the content of the disintegrating agent is 1 to 10 parts by mass based on 100 parts by mass of the magnesium oxide particles.

6. The granules according to claim 1, wherein the magnesium oxide particles are magnesium oxide particles in conformity with Japanese Pharmacopoeia.

7. The granules according to claim 1, wherein the sugar alcohol is mannitol.

8. The granules according to claim 1, wherein the disintegrating agent is carmellose.

9. The granules according to claim 1 which is a laxative agent.

Fig. 1

Dissolution test

Fig. 2

Dissolution test

1 minute after injection of sample

Sample A                    Sample X

<table>
<tr><td colspan="2">**INTERNATIONAL SEARCH REPORT**</td><td>International application No.<br>PCT/JP2010/053021</td></tr>
</table>

**A. CLASSIFICATION OF SUBJECT MATTER**
*A61K33/08*(2006.01)i, *A61K9/14*(2006.01)i, *A61K47/10*(2006.01)i, *A61K47/38*
(2006.01)i, *A61P1/10*(2006.01)i, *A61P3/02*(2006.01)i

According to International Patent Classification (IPC) or to both national classification and IPC

**B. FIELDS SEARCHED**

Minimum documentation searched (classification system followed by classification symbols)
A61K33/08, A61K9/14, A61K47/10, A61K47/38, A61P1/10, A61P3/02

Documentation searched other than minimum documentation to the extent that such documents are included in the fields searched
Jitsuyo Shinan Koho          1922-1996   Jitsuyo Shinan Toroku Koho   1996-2010
Kokai Jitsuyo Shinan Koho    1971-2010   Toroku Jitsuyo Shinan Koho   1994-2010

Electronic data base consulted during the international search (name of data base and, where practicable, search terms used)
CAP/MEDLINE/EMBASE/BIOSIS(STN), JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| Y | JP 2006-022060 A (Kyowa Chemical Industry Co., Ltd.), 26 January 2006 (26.01.2006), claims 11, 13; paragraph [0019]; examples (Family: none) | 1-9 |
| Y | JP 10-120554 A (Fuji Chemical Industry Co., Ltd.), 12 May 1998 (12.05.1998), claims; paragraphs [0005], [0007], [0008], [0012]; examples & JP 3961596 B2 | 1-9 |

☒  Further documents are listed in the continuation of Box C.          ☐  See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>12 March, 2010 (12.03.10) | Date of mailing of the international search report<br>30 March, 2010 (30.03.10) |
|---|---|
| Name and mailing address of the ISA/<br>  Japanese Patent Office | Authorized officer |
| Facsimile No. | Telephone No. |

Form PCT/ISA/210 (second sheet) (July 2009)

**INTERNATIONAL SEARCH REPORT**

| International application No. |
|---|
| PCT/JP2010/053021 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 06-065011 A (Sea Water Chemical Institute, Inc.), 08 March 1994 (08.03.1994), claims; paragraph [0015]; example 2; table 1 & EP 544502 A1 & JP 06-072816 A & US 5344636 A & EP 544502 B1 & JP 2911282 B2 & JP 2987262 B2 | 1-9 |
| Y | JP 08-048606 A (Sea Water Chemical Institute, Inc.), 20 February 1996 (20.02.1996), claims; paragraph [0009]; example 3 & JP 3736647 B2 | 1-9 |
| P,Y | WO 2009/057796 A1 (Kyowa Chemical Industry Co., Ltd.), 07 May 2009 (07.05.2009), claims; examples & TW 200924801 A | 1-9 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10120554 A

- JP 2008115058 A

**Non-patent literature cited in the description**

- Powder Property Determinations out of the General Test Processes and Apparatus of The Japanese Pharmacopoeia

- The Japanese Pharmacopoeia